Europäisches Patentamt

European Patent Office

Office européen des brevets

(19)

(11) Veröffentlichungsnummer: **0 002 495**
**B1**

(12) **EUROPÄISCHE PATENTSCHRIFT**

(45) Veröffentlichungstag der Patentschrift:
15.02.84

(21) Anmeldenummer: **78101583.9**

(22) Anmeldetag: **06.12.78**

(51) Int. Cl.³: **A 61 K 31/66** // A61K31/185 ,(A61K31/66, 31/185)

(54) **Erzeugnisse zur Anwendung in der cytostatischen Therapie.**

(30) Priorität: **14.12.77 DE 2756018**
**23.06.78 DE 2827625**

(43) Veröffentlichungstag der Anmeldung:
**27.06.79 Patentblatt 79/13**

(45) Bekanntmachung des Hinweises auf die Patenterteilung:
**15.02.84 Patentblatt 84/7**

(84) Benannte Vertragsstaaten:
**BE CH FR GB IT LU NL SE**

(56) Entgegenhaltungen:
**DE - A - 1 620 629**

(73) Patentinhaber: **Asta-Werke Aktiengesellschaft Chemische Fabrik, Artur-Ladebeck-Strasse 128 - 152, D-4800 Bielefeld 14 (DE)**

(72) Erfinder: **Brock, Norbert, Prof. Dr., Am Rehhagen 10, D-4800 Bielefeld 1 (DE)**

(74) Vertreter: **Redies, Bernd, Dr. rer. nat. et al, Redies, Redies, Türk & Gille, Patentanwälte Brucknerstrasse 20, D-4000 Düsseldorf 13 (DE)**

## Erzeugnisse zur Anwendung in der cytostatischen Therapie

Bei Oxazaphosphorin-Cytostatika wie den 2-Oxo-1,3,2-oxazaphosphorinen Cyclophosphamid, Trofosfamid, Ifosfamid und Sufosfamid treten als unerwünschte Nebenwirkung schwere Reizungen der Niere, Harnwege und/oder der Harnblase des behandelten Patienten auf. Bekannterweise treten nach erstmaliger Schädigung diese Nebenwirkungen besonders leicht auf. Diese unerwünschte Nebenwirkung kann dabei so stark sein, dass die Therapie des an Krebs erkrankten Patienten zeitweise unterbrochen oder sogar ganz unmöglich wird. Ein wesentlicher Erfolg solcher Cytostatika liegt wegen der raschen Resistenzentwicklung des Krebstumors auch in der sogenannten Stosstherapie, bei der die Cytostatika bei ihrer ersten Applikation in im Verhältnis zu ihrer Toxizität hoher Einzeldosis verabreicht werden, um eine möglichst hohe Initialkonzentration des Cytostatikums am Tumorgewebe zu erreichen. Danach werden die Cytostatika in geringen Dosen häufig über lange Zeiträume verabreicht. Es ist bekannt, dass die geschilderte unerwünschte Nebenwirkung durch im Körper des Patienten gebildete Metaboliten der Cytostatika verursacht werden. Die Nebenwirkung tritt schon bei länger bekannten Alkylantien wie unter der bekannten Bezeichnung Cyclophosphamid bekanntgewordenen 2-(N,N-Bis-(2-chloräthyl)-amino)-2-oxo-1,3,2-oxazaphosphorinan und unter dem unter der Bezeichnung Trofosfamid bekanntgewordenen 2-(N,N-Bis-(2-chloräthyl)-amino)-3-(2-chloräthyl)-2-oxo-1,3,2-oxazaphosphorinan häufig auf. Noch höhere Bedeutung hat diese Nebenwirkung, wenn man hoch cytostatisch wirksame und gleichzeitig weniger toxische Oxazaphosphorin-Cytostatika wie das Ifosfamid verabreicht. Die therapeutischen Einsatzmöglichkeiten, die aufgrund der geringen Toxizität gegeben sind, werden durch diese Nebenwirkung entscheidend eingeschänkt. Es wurde sogar beobachtet, dass aufgrund solcher Reizungen der Blase sich Blasenkrebs bildete.

Man hat viele Versuche unternommen, diese schädliche und unerwünschte Nebenwirkung der genannten Cytostatika zu beseitigen oder zumindest zu lindern, da auf deren Einsatz in der Behandlung von Krebskrankheiten nicht mehr verzichtet werden kann und ein vorzeitiger Abbruch der Therapie zu einer schweren Schädigung oder zu einem frühenTod des behandelten Patienten führt. So soll durch erhöhte Flüssigkeitsaufnahme gegebenenfalls in Verbindung mit die Urinbildung fördernden Mitteln ein möglichst rascher Durchfluss von Metaboliten der Cytostatika enthaltenem Urin durch Niere, Harnwege und Blase erreicht und die Bildung zu hoher Konzentrationen der Metaboliten insbesondere in der Blase vermieden werden. Diese sogenannte Hydratation wird im allgemeinen mit einer Alkalisierung des Urins zum Beispiel mit dem im Handel befindlichen Hexakalium-hexanatriumpentacitrat-Hydrat-Komplex und insbesondere mit der Einführung von Lösungen von Mercaptogruppen enthaltenden Verbindungen in die Blase durch Katheter verbunden. Von solchen Mercaptogruppen enthaltenden Verbindungen nahm man an, dass die Mercaptogruppe mit den alkylierenden Gruppen reagiert und so inaktiviert. Als solche Verbindung wurde insbesondere N-Acetylcystein und Cystein verwendet. Die Erfolge sind jedoch nur begrenzt, insbesondere bei Einsatz der Cytostatika in sehr hohen Dosen. Ausserdem sind Blasenspülungen für den Patienten beschwerlich und bei Verabreichung der Cytostatika über längere Zeiträume praktisch kaum durchführbar. Schliesslich sind hierdurch höher gelegene Bereiche der Harnwege überhaupt nicht erreichbar.

Eine der ersten Arbeiten, die sich mit dem Einsatz von SH-Verbindungen zur generellen Detoxifizierung bei der Cytostatika-Therapie befasst, ist T. A. Connors, Europ. J. Cancer 2, 293 bis 395 (1966). Ein Erfolg dieser Versuche blieb aus, da mit den eingesetzten SH-Verbindungen gleichzeitig der Antitumoreffekt der Cytostatika vermindert wurde (vgl. 10c, cit. S. 300 und 303, vorletzter Satz).

Als mit der Einführung der Oxazaphosphorine über das Auftreten von urotoxischen Nebenwirkungen (hämorrhagische Zysto-pyelonephritis) geklagt wurde, hat man versucht, SH-Verbindunge topisch, d.h. an den Ort der Wirkung in die Harnblase zu bringen. Diese Instillation von Acetylcystein gehört zur Standardprophylaxe gegenüber urotoxischen Nebenwirkungen bei der hochdosierten Applikation von Cyclophosphamid und Ifosfamid (vgl. z.B. Höfer-Janker, Scheef, Günther, Hüls, Med. Welt 26, 972, 1975; Drings, Fritsch, Verh. Dtsch. Ges. inn. Med. 78, 166, 1972; Cohen, Creaven, Tejada, Hansen, Muggia, Mittelmann, Selawry, Cancer Chemother. Rep. Part 1, 59, 751, 1975; Creaven, Allen, Cohen, Nelson, Cancer Treatm. Rep. 60, 445, 1976; und Primack, J. Nat. Cancer Inst. 47, 223 (1971).

Die Instillation von SH-Gruppen in die Harnblase konnte das Problem der generellen Detoxifizierung jedoch nicht lösen. Die Wirksamkeit der verwendeten SH-Verbindungen ist auf die Harnblase beschränkt. Die Praktikabilität dieser Methode (Applikation mittels Katheter) ist wenig positiv zu beurteilen. Die klinische Wirksamkeit dieser umständlichen Prophylaxe ist keineswegs befriedigend (vgl. Falkson, Suid-Afrikaanse Kankerbulletin 15, 97, 1971).

Die vorliegende Erfindung betrifft Erzeugnisse enthaltend ein Oxazaphosphorin-Cytostatikum und das Natriumsalz der 2-Mercaptoethansulfonsäure als Kombinationspräparat zur gleichzeitigen, getrennten oder zeitlich abgestuften Anwendung in der cytostatischen Therapie.

Überraschenderweise wurde gefunden, dass die geschilderte, seit langem bekämpfte schädliche Nebenwirkung von Oxazaphosphorin-Cytostatika auf die Harnwege und Blase der hiermit behandelten Menschen auf sehr einfache Weise und praktisch vollständig durch kombinierte Anwendung des lange bekannten Natriumsalzes der 2-Mercaptoethansulfonsäure in bestimmter Weise im Verhältnis zur Verabreichung des Cytostatikums erreicht wird, und zwar innerhalb eines Zeitraumes von etwa 30 Minuten vor Verabreichung des Cytostatikums bis etwa 30 Minuten nach dessen Verabreichung und in einer Menge von mindestens 20 % der zu verabreichenden bzw. verabreichten Menge des Cytostatikums bis höchstens der höchstverträglichen Dosis des Salzes der 2-Mercaptoethansulfonsäure. Das Salz der 2-Mercaptoethansulfonsäure kann dabei in der gleichen oder in einer anderen üblichen Applikationsform als das Cytostatikum verabreicht werden. Z. B. kann das Cytostatikum intravenös, das Salz der 2-Mercaptoethansulfonsäure dagegen peroral oder intravenös appliziert werden.

Es kann auch so vorgegangen werden, dass nur mindestens 25 % dieser Dosis in dieser zeitlichen Relation zur Verabreichung des Cytostatikums und die Restmengen der Gesamtdosis in einer oder mehreren Teildosen innerhalb eines Zeitraumes bis zu 12 Stunden nach Verabreichung des Cytostatikums verabreicht werden. Es wurde nämlich festgestellt, dass lediglich dafür Sorge getragen werden muss, dass im Harn, in Niere und Blase stets eine gewisse Menge an Salz der 2-Mercaptoethansulfonsäure anwesend sein muss, um ausgeschiedenes und hier auftretendes Cytostatikum bzw. eines Metaboliten hiervon unschädlich machen zu können und auf diese Weise die unerwünschte Nebenwirkung auf Niere, Harnwege und Blase wirkungsvoll zu verhindern.

Bevorzugt wird das Salz der 2-Mercaptoethansulfonsäure mit dem Cytostatikum gleichzeitig verabreicht. Dies gilt insbesondere für die in der Stosstherapie übliche hohe Initialdosis. Bevorzugt werden dann beide Verbindungen in einer einzigen Applikationseinheit verabreicht.

Um einen wirkungsvollen Schutz der mit den Cytostatika behandelten Patienten gegen die Nebenwirkung auf die Niere, Harnwege und Harnblase zu erreichen, genügt die Verabreichung schon so geringer Mengen wie 20 % der Dosis des Cytostatikums. Dies gilt insbesondere bei niedrigeren Dosen des Cytostatikums. Bei höheren Dosen des Cytostatikums kann die schädliche Nebenwirkung mit 30 % der Menge des Cytostatikums verhindert werden. Da die Nebenwirkung insbesondere bei der Verabreichung der Cytostatika in hohen Dosen von Bedeutung ist, wird die Untergrenze von 30 % der Menge des Cytostatikums bevorzugt angewendet. Im Hinblick auf die bekannte, sehr geringe Toxizität des Natriumsalzes der 2-Mercaptoethansulfonsäure ist die obere Grenze des Mengenbereichs für das Salz der 2-Mercaptoethansulfonsäure von untergeordneter Bedeutung. Überraschend und wichtig ist, dass die cytostatische Wirksamkeit der Oxazaphosphone nicht beeinträchtigt wird. Im Tierversuch wurde dies beim Ifosfamid selbst bei 100fach grösserer Dosis des Natriumsalzes der 2-Mercaptoethansulfonsäure als die Dosis des Cytostatikums festgestellt. Da im allgemeinen eine praktische Beseitigung der Nebenwirkung auch bei hohen Cytostatika-Dosen mit gleichen Mengen des Salzes der 2-Mercaptoethansulfonsäure erreicht werden, ist es bevorzugt, das Natriumsalz der 2-Mercaptoethansulfonsäure in einer Menge entsprechend 30 bis 100 % der Menge des bei Verabreichung der 2-Mercaptoethansulfonsäure vor Verabreichung des Cytostatikums zu verabreichenden bzw. des verabreichten Cytostatikums anzuwenden.

Besondere Bedeutung hat die kombinierte Verabreichung bei den in grossem Umfang zur Bekämpfung verschiedenster Krebskrankheiten eingesetzten 2-Oxo-1,3,2-oxazaphosphorinane Cyclophosphamid, Ifosfamid, Trofosfamid und Sufosfamid.

Das erfindungsgemäss angewendete Natriumsalz der 2-Mercaptoethansulfonsäure ist eine bekannte Verbindung (vgl. US-PS 2 694 733). Es ist als mucolytisch wirksame Verbindung in anderen als für die erfindungsgemässe Anwendung notwendigen Applikationsformen verwendet worden (vgl. DE-OS 1 620 629). Für die Verhinderung der geschilderten Nebenwirkung der Oxazaphosphorin-Cytostatika ist diese Verbindung bisher jedoch nicht eingesetzt worden. Bisher war man der Meinung, dass die schädliche Nebenwirkung verursachenden Oxazaphosphorin bzw. Metaboliten der Oxazaphosphorine regional am Ort der Schädigung abgefangen werden und dabei Mercaptogruppen enthaltende Verbindungen dort (z. B. durch Instillation in die Blase der behandelten Patienten) eingesetzt werden müssen, damit diese ihre Wirkung entfalten können. Dazu kommt, dass die bisher verwendeten, Mercaptogruppen enthaltenden Verbindungen in bezug auf die geschilderte Nebenwirkung sich oral appliziert als unwirksam erwiesen haben. Schliesslich war man der Meinung, dass gerade die in Betracht gezogenen Metaboliten für die cytostatische Wirksamkeit der Oxazaphosphorine verantwortlich sind und die Mercaptogruppen enthaltenden Verbindungen dementsprechend nur so spät zur Anwendung gebracht werden dürfen, dass die cytostatische Wirksamkeit nicht negativ beeinflusst wird. Selbst bei Anwendung der bisher verwendeten Mercaptogruppen enthaltenden Verbindungen ist die hierbei erreichte Wirkung im allgemeinen nur beschränkt. Die geschilderten Nebenwirkungen können auch nicht angenähert verhindert werden. Es ist nicht nur überraschend, dass das erfindungsgemäss eingesetzte Natriumsalz der 2-Mercaptoethansulfonsäuren gerade bei Applikation kurz vor, gleichzeitig oder kurz nach Verabreichung des Cytostatikums und nur dann die geschilderte schädliche Nebenwirkung vermeiden kann, sondern dass diese Verbindung diese Wirkung auch bei oraler Verabreichung in vollem Umfang entfalten.

Anwendungsbeispiele:

1. Ein 56 Jahre alter Patient, der an einem Hypernephrom der Niere leidet, wird nach den Regeln der Therapie mit Bestrahlung und Ifosfamid [2-(N-2-Chloräthylamino)-3-(2-chloräthyl)-2-oxo-1,3,2-oxazaphosphorinan] (5×60 mg/kg intravenös an 5 aufeinanderfolgenden Tagen) behandelt.

Gleichzeitig wird der Patient durch Zufuhr von 4 Liter Wasser über den Tag verteilt hydratisiert und durch Verabreichung von Hexakalium-hexanatriumpentacitrat-Hydrat-Komplex alkalisiert. Bereits am 3. Tag reagiert der Patient mit starker Makrohämaturie, die zum sofortigen Absetzen der Therapie zwingt. Nach einer Erholungspause von 14 Tagen wird die Therapie wiederholt, aber diesmal wird gleichzeitig mit jeder Dosis von 60 mg/kg Ifosfamid 35 mg/kg des Natriumsalzes der 2-Mercaptoäthansulfonsäure intravenös appliziert. Der Patient wurde täglich auf Mikro- und Makrohämaturien oder Eiweissausscheidung im Urin kontrolliert. Es waren keine Anzeichen einer Hämaturie oder Eiweissausscheidung feststellbar.

2. An einem weiteren Patienten in analoger Situation wurde die Therapie gleich zu Beginn mit kontemporärer Verabreichung der gleichen Dosis des Natriumsalzes der 2-Mercaptoäthansulfonsäure durchgeführt. Es wurden keinerlei Anzeichen einer Hämaturie festgestellt.

3. Bei zwei weiteren Patienten in analoger Situation wurden in einem Falle 21,5 mg/kg des Natriumsalzes der 2-Mercaptoäthansulfonsäure bei 60 mg/kg Einzeldosis des Cytostatikums verabfolgt, bei einem weiteren Patienten wurden 60 mg/kg des Natriumsalzes der 2-Mercapto-äthansulfonsäure bei 60 mg/kg Cytostatikums mit gleichgutem Ergebnis verabreicht.

4. Bei ähnlich gelagerten Fällen wurde das Natriumsalz der 2-Mercapto-äthansulfonsäure oral ½ Stunde vor Applikation des Cytostatikums appliziert. Bei einer Dosierung von 30 mg/kg bis 60 mg/kg des Natriumsalzes der 2-Mercaptoäthansulfonsäure auf eine Dosis von 60 mg/kg des Cytostatikums wurde ein Ausbleiben der schädlichen Nebenwirkung nachgewiesen.

Herstellungsbeispiel

5. 1. Gew.-Teil Ifosfamid (2-[N-(2-Choräthyl)-amino]-3-(2-chloräthyl)-2-oxo-1,3,2-oxazaphosphorinan und 0,63 Gew.-Teile des Natriumsalzes der 2-Mercaptoäthansulfonsäure, jeweils in reiner steriler Form, werden in einem sterilen Mischer unter aseptischen Bedingungen homogen vermischt und so in Injektionsflaschen gefüllt, dass diese auf 10 ml Injektionsflüssigkeit 500 mg Ifosfamid und 315 mg des Natriumsalzes der 2-Mercaptoäthansulfonsäure enthalten.

Nachstehend wird die überraschende Wirksamkeit der Verwendung anhand zwei verschiedener Oxazaphosphorin-Cytostatika im Tierversuchs-Modell aufgezeigt.

Die Wirksamkeit des verwendeten Natriumsalzes der 2-Mercaptoethansulfonsäuren wurde am Modell der Ifosfamid-Zystitis an der Ratte untersucht. Mit der einmaligen intravenösen Gabe von 68,1 mg/kg Ifosfamid oder Cyclophosphamid kann an der Ratte eine Zystitis ausgelöst werden. Den Tieren wird 24 Stunden nach der Zytostatikum-Gabe eine Trypanblau-Lösung intravenös appliziert, nach weiteren 30 min werden die Tiere in Äthernarkose getötet. Mit Hilfe des Vitalfarbstofes Trypanblau können Entzündungsherde gut makroskopisch dargestellt werden. Die Blasen der Versuchstiere sind tiefblau gefärbt, stark geschwollen und weisen zum Teil Blutungen auf.

Die untersuchte Verbindung wurde gleichzeitig mit dem Zytostatikum oder bis 15 min vor Applikation des Zytostatikums i.v. verabreicht.

Zur völligen Verhinderung der Zystitis bei allen Versuchstieren wurde die Dosis von 21,5 mg/kg benötigt.

Die Toxizität war sehr gering (DL50 > 2000 mg/kg).

**Patentansprüche**

1. Erzeugnisse enthaltend ein Oxazaphosphorin-Cytostatikum und das Natriumsalz der 2-Mercaptoethansulfonsäure als Kombinationspräparat zur gleichzeitigen, getrennten oder zeitlich abgestuften Anwendung in der cytostatischen Therapie.

2. Erzeugnis gemäss Anspruch 1, dadurch gekennzeichnet, dass es als Oxazaphosphorin-Cytostatikum 2-[N,N-Bis-2(-chlor-ethyl)-amino]-3-(2-chlorethyl)-2-oxo-1,3,2-oxazaphosphorinan (Trofosfamid), 2-[N-(2-chlorethyl)-amino]-3-(2-chlorethyl)-2-oxo-1,3,2-oxazaphosphorinan (Ifosfamid), 2-[N,N-Bis-(2-chlorethyl)-amino]-2-oxo-1,3,2-oxazaphosphorinan (Cyclophosphamid) oder 2-(2-Mesyloxy-ethylamino)-3-(2-chlorethyl)-2-oxo-1,3,2-oxazaphosphorinan (Sufosfamid) enthält.

3. Erzeugnisse gemäss Patentansprüchen 1 und 2, dadurch gekennzeichnet, dass sie das Natriumsalz der 2-Mercaptoethansulfonsäure und das Oxazaphosphorin-Cytostatikum in einem Gewichtsverhältnis von mindestens 0,2:1 enthalten.

**Claims**

1. Articles containing an oxazaphosphorine as cytostatically active agent and the sodium salt of 2-mercapto-ethane-sulfonic acid as combination for the simultaneous, separate or sucessive administration in the cytostatic therapy.

2. Articles according to claim 1 characterized in that they contain as cytostatically active oxazaphosphorine 2-[N,N-bis-(2-chloroethyl)-amino]-9-3-(2-chloroethyl)-2-oxo-1,3,2-oxazaphosphorinane (Trofosfamide), 2-[N-(2-chloroethyl)-amino]-3-(2-chloroethyl)-2-oxo-1,3,2-oxazaphosphorinane (Ifosfamide), 2-[N,N-bis-(2-chloroethyl)-amino]-2-oxo-1,3,2-oxazaphosphorinane (Cyclophosphamide) or 2-(2-mesyloxy-ethylamino)-3-(2-chloroethyl)-2-oxo-1,3,2-oxazaphosphorinane (Sulfosfamide).

3. Articles according to claim 1 and 2 characterized in that the sodium salt of 2-mercapto-ethan-sulfonic acid and the cytostatically active oxazaphosphorine are present in a weight proportion of at least 0.2 to 1.

**Revendications**

1. Produits contenant une oxazaphosphine cytostatique et le sel sodique de l'acide 2-mercapto-éthane-sulfonique comme préparation combinée pour utilisation simultanée, séparée ou échelonnée dans le temps en thérapeutique cytostatique.

2. Produit suivant la revendication 1, caractérisé par le fait qu'il contient comme oxazaphosphine cytostatique le 2-[N,N-bis-(2-chloréthyl)-amino]-3-(2-chloréthyl)-2-oxo-1,3,2-oxazaphosphinan (Trofosfamide), le 2-[N-(2-chloréthyl)-amino]-3-(2-chloréthyl)-2-oxo-1,3,2-oxazaphosphinan (Isosfamide), le 2-[N,N-bis-(2-chloréthyl)-amino]-2-oxo-1,3,2-oxazaphosphinan (Cyclophosphamide) ou le 2-(2-mésyloxy-éthylamino)-3-(2-chloréthyl)-2-oxo-1,3,2-oxazaphosphinan (Sulfosfamide).

3. Produits suivant les revendications 1 et 2, caractérisés par le fait qu'ils contiennent le sel sodique de l'acide 2-mercapto-éthane-sulfonique et l'oxazaphosphine cytostatique dans un rapport en poids d'au moins 0,2:1.